(19) Europäisches Patentamt
European Patent Office
Office européen des brevets

(11) Veröffentlichungsnummer: **0 256 427 B1**

(12) **EUROPÄISCHE PATENTSCHRIFT**

(45) Veröffentlichungstag der Patentschrift: **03.02.93**

(51) Int. Cl.$^5$: **C09B 67/20**, C09D 17/00, A61K 7/043, A61K 7/021, C11D 3/40, //B01F17/14, B01F17/02

(21) Anmeldenummer: **87111289.2**

(22) Anmeldetag: **05.08.87**

(54) **Pigmentdispersionen, Verfahren zu ihrer Herstellung und ihre Verwendung.**

(30) Priorität: **09.08.86 DE 3627023**

(43) Veröffentlichungstag der Anmeldung:
**24.02.88 Patentblatt 88/08**

(45) Bekanntmachung des Hinweises auf die Patenterteilung:
**03.02.93 Patentblatt 93/05**

(84) Benannte Vertragsstaaten:
**CH DE FR GB IT LI SE**

(56) Entgegenhaltungen:
**EP-A- 0 056 523**

(73) Patentinhaber: **HOECHST AKTIENGESELLSCHAFT**
**Postfach 80 03 20**
**W-6230 Frankfurt am Main 80(DE)**

(72) Erfinder: **Dietz, Erwin, Dr.**
**St.-Matthäus-Strasse 7**
**W-6233 Kelkheim (Taunus)(DE)**
Erfinder: **Münkel, Albert**
**Sindlinger Bahnstrasse 127a**
**W-6230 Frankfurt am Main 80(DE)**
Erfinder: **Memmel, Ferdinand, Dr.**
**Drosselweg 1**
**W-6230 Frankfurt am Main 80(DE)**

**EP 0 256 427 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Pigmentdispersionen, ihre Herstellung und ihre Verwendung zum Pigmentieren von wäßrigen Medien, insbesondere von Kosmetikartikeln.

Es ist eine Vielzahl von Pigmentdispersionen für die verschiedendsten Einsatzgebiete bekannt, die als Dispergiermittel anionische und/oder nichtionogene Hilfsmittel enthalten. In der Regel handelt es sich bei diesen Hilfsmitteln um Alkylaryl-Verbindungen, deren Oxalkylierungs- und/oder Sulfierungsprodukte. Diese Dispergiermittel, die sich hervorragend zum Dispergieren von Feststoffen in wäßrigen Systemen eignen, sind nur bedingt biologisch abbaubar und daher für den Einsatz auf dem Kosmetiksektor und Reinigungsmittelsektor ungeeignet. Auch sind bei diesen Produkten Haut- und Schleimhautunverträglichkeiten in der Regel nicht auszuschließen. Zum Pigmentieren von Kosmetikartikeln sind daher Pigmentdispersionen erwünscht, die neben geeigneten Farbmitteln für den Kostmetiksektor als Dispergiermittel Substanzen enthalten, die als Grundstoffe für die Kosmetik und Pharmazie Verwendung finden.

Gegenstand der vorliegenden Erfindung sind Pigmentdispersionen, bestehend aus

A) 10 bis 80 Gew.-%, vorzugsweise 20 bis 70 Gew.-% eines Pigments,

B) 2 bis 20 Gew.-%, vorzugsweise 3 bis 15 Gew.-% eines oder mehrerer Alkylglykolethersulfate der allgemeinen Formel (I)

$$R^1\text{-O-}(CH_2CH_2\text{-O-})_mSO_3M \qquad (I)$$

worin

- $R^1$ für einen Alkyl- oder Alkenylrest mit 6 bis 28 C-Atomen, vorzugsweise mit 10 bis 20 C-Atomen,
- m für eine ganze Zahl von 1 bis 20, vorzugsweise 1 bis 10 und
- M für ein physiologisch unbedenkliches Kation, vorzugsweise ein Alkalimetallkation oder ein Ammoniumion,

steht,

C) 0,1 bis 10 Gew.-%, vorzugweise 0,3 bis 5 Gew.-% eines oder mehrerer Phosphorsäureester der allgemeinen Formel (II)

$$[R^2\text{-O-}(CH_2CH_2\text{-O-})_n]_p\overset{O}{\overset{\|}{P}}(OH)_{3-p} \qquad (II)$$

worin

die $R^2$ unabhängig voneinander jeweils für einen Alkyl- oder Alkenylrest mit 4 bis 28 C-Atomen, vorzugsweise 8 bis 20 C-Atomen,

die n unabhängig voneinander jeweils für eine ganze Zahl von 0 bis 10, vorzugsweise 0 bis 5, und

p für eine ganze Zahl von 1 bis 3 stehen,

D) 0 bis 60 Gew.-%, vorzugsweise 3 bis 50 Gew.-%, insbesondere 5 bis 40 Gew.-% eines physiologisch unbedenklichen Wasserrückhaltemittels,

E) 0 bis 5 Gew.-%, vorzugsweise 0 bis 3 Gew.-% üblicher Zusatzstoffe und

F) 0 bis 80 Gew.-%, vorzugsweise 10 bis 60 Gew.-% Wasser,

wobei die Summe der enthaltenen Bestandteile (A), (B), (C), (D), (E) und (F) 100 Gew.-% der Pigmentdispersion beträgt.

Die obengenannten Bestandteile (B), (C), (D) der erfindungsgemäßen Pigmentdispersionen sind im Cosmetic-Ingredient-Dictionary in der CTFA-Liste (Cosmetic Toiletry Fragrance Association) aufgeführt. Die unter (B) aufgeführten Alkylglykolethersulfate werden in Shampoos, Schaum- und Duschbädern sowie anderen Körperreinigungsmitteln eingesetzt und sind nach der Methylenblau-Methode zu 98 % abbaubar. Die unter (C) aufgeführten Phosphorsäureester finden in der Kosmetik Verwendung als Emulgatoren in cremeförmigen Öl-in-Wasser-Emulsionen.

Läßt man bei der Herstellung der Pigmentdispersionen das unter (C) aufgeführte Hilfsmittel weg, so ist beim Dispergieren unter üblichen wäßrigen Bedingungen, beispielsweise mit Rührwerkskugelmühlen, eine extreme Schaumbildung der wäßrigen Dispersion festzustellen. Die Schaumbildung führt u.a. zu einem drastischen Rückgang der Mahlleistung der Rührwerkskugelmühlen.
Überraschenderweise gelingt es durch die Anwesenheit eines der unter (C) aufgeführten Phosphorsäureester bei der Herstellung der erfindungsgemäßen Pigmentdispersionen die übermäßige Schaumbildung auf ein normales Maß zurückzudrängen.

Die nach dem Dispergieren erhaltenen wäßrigen Pigmentdispersionen können als solche in Anwendungsmedien eingearbeitet oder auch getrocknet und als Pulver weiterverarbeitet werden.

Die erfindungsgemäßen Pigmentdispersionen weisen überraschend hohe Farbstärke und gute Lagerstabilität auf. Der Bestandteil (C) hat dabei keinen negativen Einfluß auf die genannten anwendungstechnischen Eigenschaften der Pigmentdispersionen.

Von besonderem Interesse sind die erfindungsgemäßen Pigmentdispersionen, bei denen der Bestandteil (B) aus folgenden Verbindungen ausgewählt ist:
Alkalisalze und Ammoniumsalze, vorzugsweise Natriumsalze von Alkyl- oder Alkenylmonoglykolethersulfaten oder Alkyl- oder Alkenyloligoglykolethersulfaten mit bis zu 20, vorzugsweise bis zu 10, insbesondere 2 oder 3 Oxethyleinheiten und mit Alkylresten bzw. Alkenylresten wie Hexyl, Heptyl, Octyl, Nonyl, Decyl, Undecyl, Dodecyl, Tetradecyl, Pentadecyl, Hexadecyl, Heptadecyl, Octadecyl, Nonadecyl, Eicosyl, Heneicosyl, Docosyl, Tricosyl, Tetracosyl, Pentacosyl, Hexacosyl, Heptacosyl, Octacosyl, 2-Methyl-pentyl, 2-Ethyl-hexyl, 2-Propyl-heptyl, 2-Butyl-octyl, 2-Pentyl-nonyl, 2-Hexyl-decyl, 2-Heptyl-undecyl, 2-Octyl-dodecyl, 2-Nonyl-tridecyl, 2-Decyl-tetradecyl, 2-Undecyl-pentadecyl, 2-Dodecyl-hexadecyl, 10-Undecenyl, 9c-Octadecenyl, 9t-Octadecenyl, 9c,12c-Octadecadienyl, 9c, 12c, 15c-Octadecatrienyl, 9c-Eicosenyl, 5,8,11,14-Eicosatetraenyl, 13c-Docosenyl und 13t-Docosenyl, vorzugsweise Decyl, Dodecyl, Tetradecyl, Hexadecyl, Octadecyl und 9c-Octadecenyl; besonders bevorzugt sind als Bestandteile (B) der erfindungsgemäßen Pigmentdispersionen die Natriumsalze von Oleyltriglykolethersulfat, Stearyltriglykolethersulfat, Cetyltriglykolethersulfat, Tetradecyltriglykolethersulfat Lauryltriglykolethersulfat, Lauryldiglykolethersulfat, Tetradecyldiglykolethersulfat, Cetyldiglykolethersulfat, Oleyldiglykolethersulfat, Stearyldiglykolethersulfat und Gemische der genannten Verbindungen, insbesondere Lauryldiglykolethersulfat und Tetradecyldiglykolethersulfat.

Die erfindungsgemäßen Pigmentdispersionen enthalten als Bestandteil (C) Orthophosphorsäureester von gegebenenfalls mit bis zu 10 Mol Ethylenoxid, vorzugsweise mit bis zu 5 Mol Ethylenoxid oxethylierten Alkanolen oder Alkenolen wie Butanol, Pentanol, Hexanol, Heptanol, Octanol, Nonanol, Decanol, Undecanol, Dodecanol, Tridecanol, Tetradecanol, Pentadecanol, Hexadecanol, Heptadecanol, Octadecanol, Nonadecanol, Eicosanol, Heneicosanol, Docosanol, Tricosanol, Tetracosanol, Pentacosanol, Hexacosanol, Heptacosanol, Octacosanol, 2-Methyl-pentanol, 2-Ethyl-hexanol, 2-Propylheptanol, 2-Butyl-octanol, 2-Pentyl-nonanol, 2-Hexyl-decanol, 2-Heptyl-undecanol, 2-Octyl-dodecanol, 2-Nonyl-tridecanol, 2-Decyl-tetradecanol, 3-Undecyl-pentadecanol, 2-Dodecyl-hexadecanol, 10-Undecenol, 9c-Octadecenol, 9t-Octadecenol, 9c,12c-Octadecadienol, 9c,12c,15c-Octadecatrienol, 9c-Eicosenol, 5,8,11,14-Eicosatetraenol,
13c-Docosenol und 13t-Docosenol, vorzugsweise Mono-, Di- und Tri-Orthophosphorsäureester von Octanol, Decanol, Dodecanol, Tetradecanol, Hexadecanol, Octadecanol, 9c-Octadecanol, Eicosanol und Oxethylate der Alkohole mit bis zu 5 Oxethyl-Einheiten sowie Gemische der Orthophosphorsäureester. Besonders bevorzugte Phosphorsäureester der obengenannten Formel (II) sind Mono-, Di- und Tri-(lauryltetraglykolether)-orthophosphorsäureester, Mono-, Di- und Tri-(tetradecyltetraglykolether)-orthophosphorsäureester, Mono-, Di- und Tri- (cetyltetraglykol)-orthophosphorsäureester, Mono-, Di- und Tri-(stearyltetraglykolether)-orthophosphorsäureester, Mono-, Di- und Tri-(octadecenylglykolether)-orthophosphorsäureester und besonders Mono-, Di- und Tri-(octadecenyl)-orthophosphorsäureester.

Als Bestandteil (D) der erfindungsgemäßen Pigmentdispersionen eignen sich vorzugsweise Wasserrückhaltemittel des Zuckeralkoholtyps, wie Sorbit, Mannit, Adonit, Dulcit, Erthrit, Xylith und insbesondere Glycerin, oder Glykole wie 1,2-Propandiol.

Die erfindungsgemäßen Pigmentdispersionen können außerdem andere für den Kosmetiksektor übliche Hilfsstoffe wie Antiabsetzmittel, Netzmittel und Konservierungsmittel in untergeordneten Mengen enthalten (siehe Bestandteil( E)).

Die erfindungsgemäßen Pigmentdispersionen enthalten als Bestandteil (A) Pigmente, wobei hier unter dem Begriff Pigment weitgehend in Wasser und in organischen Lösemitteln unlösliche Farbmittel, wie anorganische Pigmente, Pigment-Füllstoffe und organische Pigmente, zu verstehen sind, soweit sie in der Kosmetik-Verordnung zugelassen sind.

Als anorganische Pigmente bzw. Füllstoffe eignen sich beispielsweise Weißpigmente und Buntpigmente wie Titandioxide, Eisenoxide und Ruß, insbesondere Pigment Yellow 42/43 (C.I. Nr. 77492), Pigment Brown 6/7 (C.I. Nr. 77491), Pigment Black 11 (C.I. Nr. 77499) und Pigment White 6 (C.I. Nr. 77891).

Als geeignete organische Pigmente sind u.a. Pigmente aus der Gruppe der Azo-, Kupferphthalocyanin-, Indigo-, Anthrachinon-, Dioxazin- und Chinacridon-Pigmente zu nennen, vorzugsweise Pigment Red 4 (C.I. Nr. 12085), Pigment Red 181 (C.I. Nr. 73360), Pigment Red 5 (C.I. Nr. 12490), Pigment Red 68 (C.I. Nr. 15525), Pigment Green 7 (C.I. Nr. 74260), Pigment Red 3 (C.I. Nr. 12120), Pigment Red 12 (C.I. Nr. 12385), Pigment Blue 15 (C.I. Nr. 74160), aber auch beispielsweise Pigment Yellow 1 (C.I. Nr. 11680), Pigment Orange 43 (C.I. Nr. 71105), Pigment Red 2 (C.I. Nr. 12310), Pigment Red 7 (C.I. Nr. 12420), Pigment Red 8 (C.I. Nr. 12335), Pigment Red 10 (C.I. Nr. 12440), Pigment Violet 23 (C.I. Nr. 51319), Pigment Red 209 (C.I.

Nr. 73905), Pigment Yellow 12 (C.I. Nr. 21090), Pigment Yellow 13 (C.I. Nr. 21100), Pigment Yellow 83 (C.I. Nr. 21108), Pigment Orange 34 (C.I. Nr. 21115), Pigment Red 9 (C.I. Nr. 12460) und Pigment Red 112 (C.I. Nr. 12370), Pigment Orange 4 (C.I. Nr. 12459), Pigment Orange 1 (C.I. Nr. 11725), Pigment Red 146 (C.I. Nr. 12485), Pigment Red 171 (C.I. Nr. 12512), Pigment Red 175 (C.I. Nr. 12513), Pigment Red 88 (C.I. Nr. 73312), Pigment Red 209 (C.I. Nr. 73905), Pigment Red 122, Pigment Reds 144, 166, 170 and 188, Pigment Orange 13 (C.I. Nr. 21110), Pigment Orange 20 (C.I. Nr. 77199), Pigment Yellow 2 (C.I. Nr. 11730), Pigment Yellow 49 (C.I. Nr. 11765), Pigment Yellow 97 (C.I. Nr. 11767), Pigment Yellow 16 (C.I. Nr. 20040), Pigment Yellow 17 (C.I. Nr. 21105), Pigment Yellow 55 (C.I. Nr. 21096), Pigment Yellow 38 (C.I. Nr. 77878), Pigment Yellow 36 (C.I. Nr. 77975), Pigment Green 8 (C.I. Nr. 10006), Pigment Green 10 (C.I. Nr. 12775), Pigment Green 37 (C.I. Nr. 74255), Pigment Blue 16 (C.I. Nr. 74100) und Pigment Blue 30 (C.I. Nr. 77420).

Gegenstand der Erfindung ist auch das Verfahren zur Herstellung der erfindungsgemäßen Pigmentdispersionen, wobei die Komponente (A) in Form von Pulver, Granulat oder wäßrigem Preßkuchen in Gegenwart von Wasser sowie mindestens den weiteren Komponenten (B) und (C) in an sich üblicher Weise dispergiert wird, anschließend die gegebenenfalls übrige Komponente (D) und/oder (E) zugemischt und die erhaltene wäßrige Pigmentdispersion mit Wasser auf die gewünschte Konzentration eingestellt oder die wäßrige Pigmentdispersion getrocknet wird.

Vorzugsweise werden die Komponenten (B), (C), (D), (E) und (F) zunächst vermischt, die Komponente (A) in die vorgelegte Mischung eingerührt und je nach Kornhärte der eingesetzten Feststoffe, beispielsweise mit Rührwerken, Dissolvern, Rotor-Stator-Mühlen, Kugelmühlen, Rührwerkskugelmühlen wie Sand- und Perlmühlen, Schnellmischern oder Knetappataturen dispergiert.

Getrocknete Pigmentdispersionen werden vorzugsweise ohne Wasserrückhaltemittel (Komponente D) hergestellt. Die Trocknung erfolgt dabei in für wäßrige Pigmentdispersionen üblicher Weise, insbesondere mittels Sprühtrocknung.

Gegenstand der Erfindung ist auch die Verwendung der erfindungsgemäßen Pigmentdispersion zum Pigmentieren von wäßrigen Medien, insbesondere von Kosmetikartikeln.

Erfindungsgemäße Pigmentdispersion besitzen eine gute Lagerstabilität und einen hohen Feststoffgehalt. Die wäßrigen Pigmentdispersionen besitzen gute rheologische Eigenschaften. Die wäßrigen und die trockenen Pigmentdispersionen eignen sich zum Pigmentieren von wäßrigen Medien, insbesondere von Kosmetikartikeln, wie Seifen, Fingermalfarben und Waschpulvern, sowie Mitteln für die dekorative Kosmetik, wie Cremes, Make-up und Schminkfettstifte.

In den folgenden Herstellungsbeispielen bezeichnen Teile Gewichtsteile; Prozentangaben und Verhältnisse beziehen sich auf das Gewicht.

Beispiel 1

In einem Mahlbehälter wurden 8 Teile einer Mischung aus Mono-, Di- und Tri-oleyl-orthophosphorsäureester sowie 28 Teile einer 70 %igen wäßrigen Lösung eines Alkyldiglykolethersulfat-Natriumsalzes (Alkyl = $C_{12}/C_{14}$-Alkyl im Verhältnis von ca. 72 : 28; nativ), 40 Teile Glycerin und 85 Teile Wasser vorgelegt und gemischt. Nachdem am Dissolver 200 Teile Pigment Yellow 3 eingerührt wurden, fügte man 1200 Teile Siliquarzit-Perlen (⌀ 1 mm) zu und ließ 1 Stunde in einer diskontinuierlichen Rührwerkskugelmühle mahlen. Bei einer Kühlwassertemperatur von 10° C betrug die Mahlguttemperatur 30° C. Das Mahlgut wurde nach Verdünnen mit 39 Teilen Wasser von den Mahlkörpern abgetrennt und entlüftet. Man erhielt eine lagerstabile, sehr gut fließfähige Pigmentdispersion mit einem Pigmentgehalt von 50 %.

Beispiel 2

Analog Beispiel 1 wurden 180 Teile Pigment Yellow 1 in einer Mischung aus 24 Teilen der 70 %igen wäßrigen Lösung des Alkyldiglykolethersulfat-Natriumsalzes aus Beispiel 1, 12 Teilen einer Mischung aus Mono-, Di- und Tri-(alkyltetraglykolether)-Orthophosphorsäureester (Alkyl = $C_{12}/C_{14}$-Alkyl, nativ), 40 Teilen Sorbit und 113 Teilen Wasser in einer Perlmühle dispergiert.
Nach Verdünnen mit 31 Teilen Wasser erhielt man eine gut fließfähige, lagerstabile Pigmentdispersion mit einem Pigmentgehalt von 45 %.

Beispiel 3

Analog Beispiel 1 wurden 160 Teile Pigment Blue 15:1 (C.I. Nr. 74160) in einer Mischung aus 32 Teilen einer 70%igen wäßrigen Lösung eines Alkyltriglykolethersulfat-Natriumsalzes (Alkyl = $C_{12}/C_{14}$-Alkyl; synthetisch), 8 Teilen einer Mischung aus Mono, Di- und Trioleylorthophosphorsäureester, 80 Teilen Glycerin

und 80 Teilen Wasser in einer Perlmühle dispergiert. Nach Verdünnen mit 40 Teilen Wasser erhielt man eine gut fließfähige, lagerstabile Pigmentdispersion mit einem Pigmentgehalt von 40 %.

Beispiel 4

Analog Beispiel 1 wurden 235 Teile Pigment Green 7 in einer Mischung aus 37,5 Teilen des in Beispiel 1 beschriebenen Alkyldiglykolethersulfat-Natriumsalzes, 15 Teilen des in Beispiel 1 beschriebenen Phosphorsäureesters, 125 Teilen Glycerin und 67,5 Teilen Wasser dispergiert und mit 20 Teilen Wasser verdünnt. Die Viskosität der erhaltenen Pigmentdispersion betrug 0,83 Pas (Pas = Pascalsekunden) bei einem Schergefälle von D = 56 $s^{-1}$.

Vergleichsbeispiel 1

Analog Beispiel 1 wurden 240 Teile Pigment Green 7 in einer Mischung aus 37,5 Teilen des in Beispiel 1 beschriebenen Alkyldiglykolethersulfat-Natriumsalzes, 125 Teilen Glycerin und 75 Teilen Wasser dispergiert und anschließend mit 22,5 Teilen Wasser verdünnt. Die Viskosität der erhaltenen Pigmentdispersion betrug 0,85 Pas bei einem Schergefälle von D = 56 $s^{-1}$. Während der Dispergierung in der Perlmühle trat starke Schaumbildung auf.

Vergleichsbeispiel 2

Analog Beispiel 1 wurden 230 Teile Pigment Green 7 in einer Mischung aus 30 Teilen eines Additionsprodukts von Nonylphenol und 30 Mol Ethylenoxid, 150 Teilen Glycerin und 80 Teilen Wasser dispergiert. Nach Verdünnen mit 35 Teilen Wasser betrug die Viskosität der erhaltenen Pigmentdispersion 0,82 Pas bei einem Schergefälle von D = 56 $s^{-1}$.

Schaumtest

Um das Schaumverhalten unter Praxis- bzw. Herstellungbedingungen zu prüfen, wurden die Pigmentpräparationen aus Beispiel 4, Vergleichsbeispiel 1 und Vergleichsbeispiel 2 bei konstanten Bedingungen intensiv gerührt. Die Rührzeit mit einem Sägezahndissolver (n = 5000 $min^{-1}$; Produktmenge 200 g; Scheibendurchmesser 30 mm) betrug jeweils 30 min. Die Pigmentdispersionen besaßen vergleichbare Viskosität. Als Maß für die Schaumbildung wurde die Dichte der Dispersion nach dem Rühren gemessen und mit der Dichte nach Entlüften der Dispersion vergleichen. Die gemessenen Dichten sind zusammen mit den Viskositäten der entlüfteten Pigmentdispersionen in der Tabelle 1 aufgeführt.

Tabelle 1

| Dispersion | Viskosität D= 56 $s^{-1}$ (Pas) | Dichte entlüftet (g x $cm^{-3}$) | Dichte nach dem Rühren (g x $cm^{-3}$) |
|---|---|---|---|
| Beispiel 4 | 0,83 | 1,45 | 0,57 |
| Vergleichsbeispiel 1 | 0,85 | 1,47 | 0,35 |
| Vergleichsbeispiel 2 | 0,82 | 1,40 | 0,56 |

Wie in Tabelle 1 zu sehen ist, neigt die Dispersion aus Vergleichsbeispiel 1 ohne Phosphorsäureester extrem zur Schaumbildung, was aus der geringen Dichte nach dem Rühren hervorgeht. Vergleichsbeispiel 2, das ein handelsübliches nichtionogenes Dispergiermittel enthält, welches nur ungenügend biologisch abbaubar ist, ist hinsichtlich der Schaumneigung mit dem erfindungsgemäßen Beispiel 4 vergleichbar.

Anwendungsbeispiele

100 Teile Seifengrundmasse werden in einem Kneter vorgelegt, 0,2 Teile der in den Beispielen 1, 2, 3 oder 4 beschriebenen Pigmentpräparation sowie 50 Teile Wasser zugesetzt und 30 Minuten geknetet. Die gefärbte Seifengrundmasse wird anschließend bei 40 °C in einer Strangpresse erwärmt und nach Austritt in einer Form verpreßt. Man erhält farbstarke, transparente Formstücke. Deckende Färbungen erhält man, wenn zusätzlich zur Pigmentpräpration 2 Teile Titandioxid eingeknetet werden.

**Patentansprüche**

**1.** Pigmentdispersion, bestehend aus
A) 10 bis 80 Gew.-% eines Pigments,
B) 2 bis 20 Gew.-% eines oder mehrerer Alkylglykolethersulfate der allgemeinen Formel (I),

$R^1\text{-O-}(CH_2CH_2\text{-O-})_mSO_3M$ (I)

worin
$R^1$ für einen Alkyl- oder Alkenylrest mit 6 bis 28 C-Atomen,
m für eine ganze Zahl von 1 bis 20 und
M für ein physiologisch unbedenkliches Kation steht,
C) 0,1 bis 10 Gew.-% eines oder mehrerer Phosphorsäureester der allgemeinen Formel (II),

$$[R^2\text{-O-}(CH_2CH_2\text{-O-})_n]_p\overset{O}{\overset{\|}{P}}\text{-}(OH)_{3-p} \quad \text{(II)}$$

worin
die $R^2$ unabhängig voneinander jeweils für einen Alkyl- oder Alkenylrest mit 4 bis 28 C-Atomen,
die n unabhängig voneinander jeweils für eine ganze Zahl von 0 bis 10 und
p für eine ganze Zahl von 1 bis 3 stehen,
D) 0 bis 60 Gew.-% eines physiologisch unbedenklichen Wasserrückhaltemittels,
E) 0 bis 5 Gew-% üblicher Zusatzstoffe und
F) 0 bis 80 Gew.-% Wasser,
wobei die Summe der enthaltenen Bestandteile (A), (B), (C), (D), (E) und (F) 100 Gew.-% der Dispersion beträgt.

**2.** Pigmentdispersion nach Anspruch 1, gekennzeichnet durch einen Gehalt von
(A) 20 bis 70 Gew.-% eines Pigments,
(B) 3 bis 15 Gew.-% eines oder mehrerer der im Anspruch 1 genannten Alkylglykolethersulfate der Formel (I),
(C) 0,3 bis 5 Gew.-% eines oder mehrerer der in Anspruch 1 genannten Phosphorsäureester der allgemeinen Formel (II),
(D) 3 bis 50 Gew.-% eines Wasserrückhaltemittels,
(E) 0 bis 3 Gew.-% üblicher Zusatzstoffe,
(F) 10 bis 60 Gew.-% Wasser,
wobei die Summe der enthaltenen Bestandteile (A) bis (F) 100 Gew.-% der Dispersion beträgt.

**3.** Pigmentdispersion nach Anspruch 1 oder 2, dadurch gekennzeichnet, daß ein oder mehrere Alkylglykolethersulfate der in Anspruch 1 genannten Formel (I), worin
$R^1$ für einen Alkyl- oder Alkenylrest mit 10 bis 20 C-Atomen,
m für eine Zahl von 1 bis 10 und

M für ein Alkalimetall oder das Ammoniumion steht,
enthalten ist bzw. sind.

4. Pigmentdispersion nach Anspruch 3, dadurch gekennzeichnet, daß $R^1$ für einen Alkyl- oder Alkenylrest mit 12 bis 18 C-Atomen und
   m für 2 oder 3 steht.

5. Pigmentdispersion nach einem oder mehreren der Ansprüche 1 bis 4, dadurch gekennzeichnet, daß ein oder mehrere Phosphorsäureester der in Anspruch 1 genannten allgemeinen Formel (II), worin
   $R^2$ für einen Alkyl- oder Alkenylrest mit 8 bis 20 C-Atomen,
   n für 0, 1, 2, 3, 4 oder 5 und
   p für 1, 2 oder 3 steht,
   enthalten ist bzw. sind.

6. Pigmentdispersion nach Anspruch 5, dadurch gekennzeichnet, daß $R^2$ für einen Alkyl- oder Alkenylrest mit 12 bis 18 C-Atomen steht.

7. Verfahren zur Herstellung der nach einem der Ansprüche 1 bis 6 definierten Pigmentdispersionen, dadurch gekennzeichnet, daß die Komponente (A) in Gegenwart von Wasser und mindestens den weiteren Komponenten (B) und (C) dispergiert wird, anschließend die gegebenenfalls übrige Komponente (D) und/oder (E) zugemischt und die erhaltene wäßrige Pigmentdispersion mit Wasser auf die gewünschte Konzentration eingestellt oder die wäßrige Pigmentdispersion getrocknet wird.

8. Verwendung einer nach einem oder mehreren der Ansprüche 1 bis 6 definierten Pigmentdispersionen zum Pigmentieren von wäßrigen Medien.

9. Verwendung nach Anspruch 8 zum Pigmentieren von Kosmetikartikeln.

10. Verwendung nach Anspruch 9 zum Pigmentieren von Seifen, Fingermalfarben und Waschpulvern.

## Claims

1. A pigment dispersion comprising
   A) 10 to 80% by weight of a pigment,
   B) 2 to 20% by weight of one or more alkylglycol ether-sulfates of the formula (I)

   $$R^1\text{-O-}(CH_2CH_2\text{-O-})_mSO_3M \qquad (I)$$

   in which
   $R^1$ represents an alkyl or alkenyl radical with 6 to 28 carbon atoms,
   m represents an integer from 1 to 20 and
   M represents a physiologically acceptable cation,
   C) 0.1 to 10% by weight of one or more phosphoric acid esters of the formula (II)

   $$[R^2\text{-O-}(CH_2CH_2\text{-O-})_n]_p\overset{O}{\overset{\|}{P}}\text{-}(OH)_{3-p} \qquad (II)$$

   in which
   the radicals $R^2$ independently of one another each represent an alkyl or alkenyl radical with 4 to 28 carbon atoms,
   the symbols n independently of one another each represent an integer from 0 to 10 and
   p represents an integer from 1 to 3,
   D) 0 to 60% by weight of a physiologically acceptable water retention agent,
   E) 0 to 5% by weight of customary additives and
   F) 0 to 80% by weight of water,

the sum of the constituents (A), (B), (C), (D), (E) and (F) present making up 100% by weight of the dispersion.

**2.** A pigment dispersion as claimed in claim 1, which contains
(A) 20 to 70% by weight of a pigment,
(B) 3 to 15% by weight of one or more of the alkylglycol ether-sulfates of the formula (I) mentioned in claim 1,
(C) 0.3 to 5% by weight of one or more of the phosphoric acid esters of the formula (II) mentioned in claim 1,
(D) 3 to 50% by weight of a water retention agent,
(E) 0 to 3% by weight of customary additives,
(F) 10 to 60% by weight of water,
the sum of the constituents (A) to (F) present making up 100% by weight of the dispersion.

**3.** A pigment dispersion as claimed in either of claims 1 or 2, which contains one or more alkylglycol ether-sulfates of the formula (I) mentioned in claim 1, in which
$R^1$ represents an alkyl or alkenyl radical with 10 to 20 carbon atoms,
m represents a number from 1 to 10 and
M represents an alkali metal or the ammonium ion.

**4.** A pigment dispersion as claimed in claim 3, wherein
$R^1$ represents an alkyl or alkenyl radical with 12 to 18 carbon atoms and
m represents 2 or 3.

**5.** A pigment dispersion as claimed in one or more of claims 1 to 4, which contains one or more phosphoric acid esters of the formula (II) given in claim 1, in which
$R^2$ represents an alkyl or alkenyl radical with 8 to 20 carbon atoms,
n represents 0, 1, 2, 3, 4 or 5 and
p represents 1, 2 or 3.

**6.** A pigment dispersion as claimed in claim 5, wherein $R^2$ represents an alkyl or alkenyl radical with 12 to 18 carbon atoms.

**7.** A process for the preparation of a pigment dispersion as defined in any one of claims 1 to 6, which comprises dispersing component (A), in the presence of water and at least the other components (B) and (C), subsequently admixing the other optional components (D) and/or (E) and adjusting the resulting aqueous pigment dispersion to the desired concentration with water or drying the aqueous pigment dispersion.

**8.** The use of a pigment dispersion as defined in one or more of claims 1 to 6 for pigmenting an aqueous medium.

**9.** The use as claimed in claim 8, for pigmenting cosmetic articles.

**10.** The use as claimed in claim 9, for pigmenting soaps, fingerpaint colors and washing powders.

### Revendications

**1.** Dispersion pigmentaire constituée :
A) de 10 à 80 % en poids d'un pigment,
B) de 2 à 20 % en poids d'un ou plusieurs sulfates d'éthers alkyliques de glycols répondant à la Formule générale I :

$$R^1\text{-O-}(CH_2CH_2\text{-O-})_mSO_3M \quad (I)$$

dans laquelle
$R^1$ représente un radical alkyle ou alcényle contenant de 6 à 28 atomes de carbone,
m désigne un nombre entier de 1 à 20, et

M représente un cation sans inconvénient du point de vue physiologique,

C) de 0,1 à 10 % en poids d'un ou plusieurs esters phosphoriques répondant à la Formule Générale II :

$$[R^2\text{-O-}(CH_2CH_2\text{-O-})_n]_p\overset{O}{\overset{\|}{P}}\text{-}(OH)_{3-p} \qquad \text{(II)}$$

dans laquelle

les $R^2$ représentent chacun, indépendamment l'un de l'autre , un radical alkyle ou alcényle contenant de 4 à 28 atomes de carbone,

les n représentent chacun, indépendamment l'un de l'autre, un nombre entier de 0 à 10, et

p désigne un nombre entier de 1 à 3,

D) de 0 à 60 % en poids d'un agent de rétention de l'eau sans inconvénient du point de vue physiologique,

E) de 0 à 5 % en poids d'additifs usuels, et

F) de 0 à 80 % en poids d'eau,

la somme des teneurs en les constituants (A), (B), (C), (D), (E) et (F) représentant 100 % en poids de la dispersion.

**2.** Dispersion pigmentaire selon la revendication 1 caractérisée en ce qu'elle contient :

A) de 20 à 70 % en poids d'un pigment,

B) de 3 à 15 % en poids d'un ou plusieurs sulfates d'éthers alkyliques de glycols de Formule I tels que spécifiés à la revendication 1,

C) de 0,3 à 5 % en poids d'un ou de plusieurs esters phosphoriques de Formule générale II tels que spécifiés à la revendication 1,

D) de 3 à 50 % en poids d'un agent de rétention d'eau,

E) de 0 à 3 % en poids d'additifs usuels, et

(F) de 10 à 60 % en poids d'eau,

la somme des teneurs en les constituants (A) à (F) représentant 100 % en poids de la dispersion.

**3.** Dispersion pigmentaire selon l'une des revendications 1 et 2 caractérisée en ce qu'elle contient un ou plusieurs sulfates d'éthers alkyliques de glycols qui répondent à la Formule I représentée à la revendication 1 dans laquelle

$R^1$ représente un radical alkyle ou alcényle contenant de 10 à 20 atomes de carbone,

m désigne un nombre de 1 à 10, et

M désigne un métal alcalin ou un ion d'ammonium.

**4.** Dispersion pigmentaire selon la revendication 3 caractérisée en ce que :

$R^1$ représente un radical alkyle ou alcényle contenant de 12 à 18 atomes de carbone, et

m est égal à 2 ou à 3.

**5.** Dispersion pigmentaire selon l'une quelconque des revendications 1 à 4, caractérisée en ce qu'elle contient un ou plusieurs esters phosphoriques répondant à la Formule générale II représentée à la revendication 1 dans laquelle

$R^2$ représente un radical alkyle ou alcényle contenant de 8 à 20 atomes de carbone,

n est égal à 0, à 1, à 2, à 3, à 4 ou à 5 et

p est égal à 1, à 2 ou à 3.

**6.** Dispersion pigmentaire selon la revendication 5 caractérisée en ce que $R^2$ représente un radical alkyle ou alcényle contenant de 12 à 18 atomes de carbone.

**7.** Procédé pour préparer des dispersions pigmentaires selon l'une quelconque des revendications 1 à 6, procédé caractérisé en ce qu'on disperse la composante (A) en présente d'eau et d'au moins les autres composantes (B) et (C), puis on ajoute les éventuelles autres composantes (D) et/ou (E) et on ajuste la dispersion pigmentaire aqueuse obtenue, avec de l'eau, à la concentration souhaitée ou on sèche la dispersion pigmentaire aqueuse.

**8.** Application d'une dispersion pigmentaire telle que spécifiée dans l'une quelconque des revendications 1 à 6 à la pigmentation de milieux aqueux.

**9.** Application selon la revendication 8 pour la pigmentation de produits cosmétiques.

**10.** Application selon la revendication 9 pour la pigmentation de savons, de vernis à ongle et de poudres détergentes.